(19) Europäisches Patentamt
European Patent Office
Office européen des brevets

(11) Numéro de publication : **0 025 740**
**B1**

(12) # FASCICULE DE BREVET EUROPÉEN

(45) Date de publication du fascicule du brevet : **13.01.82**

(51) Int. Cl.³ : **C 07 C 33/02**, C 07 C 29/44

(21) Numéro de dépôt : **80401245.8**

(22) Date de dépôt : **01.09.80**

(54) **Nouveaux alcools gras insaturés et leur procédé de préparation.**

(30) Priorité : **14.09.79 FR 7922976**

(43) Date de publication de la demande : **25.03.81 (Bulletin 81/12)**

(45) Mention de la délivrance du brevet : **13.01.82 Bulletin 82/02**

(84) Etats contractants désignés : **DE FR GB IT NL**

(56) Documents cités :
**DE - A - 2 148 156**
**DE - B - 1 139 488**
**FR - A - 2 039 652**
**FR - A - 2 045 369**
**FR - A - 2 266 682**
**US - A - 3 887 627**

(73) Titulaire : **RHONE-POULENC INDUSTRIES**
**Brevets Pharma 25 Quai Paul Doumer**
**F-92408 Courbevoie Cedex (FR)**

(72) Inventeur : **Morel, Didier**
**19, avenue Jean Mermoz**
**F-69008 Lyon (FR)**

(74) Mandataire : **Cazes, Jean Marie et al**
**Rhône-Poulenc Service Brevets Chimie B.P.753**
**F-75360 Paris Cedex 08 (FR)**

**EP 0 025 740 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Nouveaux alcools gras insaturés et leur procédé de préparation

La présente invention a pour objet de nouveaux alcools gras insaturés : elle concerne également la préparation de ces alcools gras insaturés.

Les composés selon l'invention ont pour formule générale

(A) 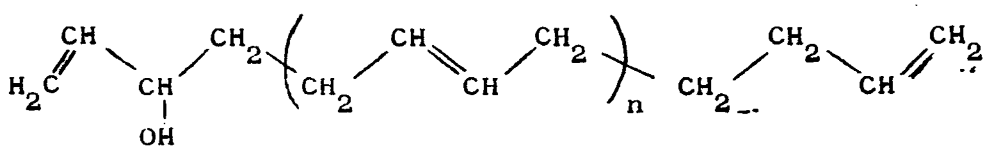

dans laquelle n est égal à 2 ou 4.

L'invention concerne donc plus particulièrement l'hexadécatétraène-1,6,10,15 ol-3 (n = 2) et le tétracosahexaène-1,6,10,14,18,23 ol-3 (n = 4).

L'invention a également pour objet un procédé de préparation des composés de formule A caractérisé en ce qu'on fait réagir du butadiène avec de l'eau en présence d'un sel de palladium et d'acide orthoborique $B(OH)_3$ dans un solvant aprotique polaire.

Selon un mode de réalisation particulier de l'invention, on met en œuvre dans le solvant aprotique polaire, simultanément au sel de palladium et à l'acide orthoborique, un acide carboxylique.

Il doit être souligné que, dans le cadre de la présente invention, l'ajout d'acide carboxylique n'est pas impératif. Il permet cependant d'augmenter la stabilité du système catalytique constitué à partir du sel de palladium ; il permet également d'augmenter la sélectivité en alcool (A) où n = 2.

Le sel de palladium est choisi de préférence parmi le groupe comprenant les carboxylates de palladium de formule $(R_1COO)_2$ Pd(B), les complexes allyliques du palladium possédant un radical carboxylate de formule $(R_2$ Pd $OOCR_1)_2$ (C) et les complexes allyliques du palladium de formule $R_2$ Pd O $R_1$ (D) où :

$R_1$ représente un radical choisi parmi le groupe comprenant les radicaux alkyle ayant de 1 à 6 atomes de carbone comme les radicaux méthyle, éthyle, isopropyle, n-butyle et les radicaux aryle comme les radicaux phényle, tolyle, benzyle, naphtyle, ces radicaux pouvant être éventuellement substitués.

$R_2$ représente un radical de formule

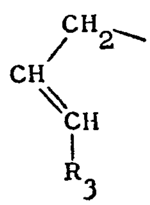

où

$R_3$ représente un atome d'hydrogène ou le radical $R_1$.

On peut citer comme exemples de carboxylates de palladium (B) utilisables dans le procédé selon l'invention l'acétate de palladium et le benzoate de palladium.

On peut citer comme exemples de complexes allyliques de palladium (C) utilisables dans le procédé selon l'invention les composés suivants :

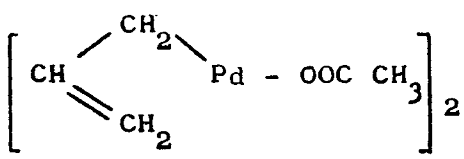 ; 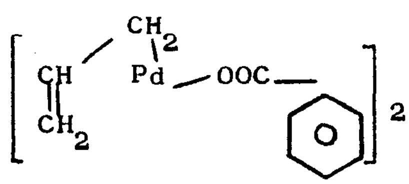

On peut citer comme exemple de complexe D :

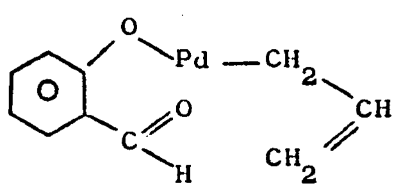

Le solvant est choisi de préférence parmi le groupe comprenant la diméthylformamide, l'hexaméthylphosphore-triamide, la diméthylacétamide, la N-méthylpyrrolidone.

L'acide carboxylique éventuellement utilisé dans le cadre de la présente invention a pour formule générale $R_4$ COOH dans laquelle $R_4$ représente un radical choisi parmi le groupe comprenant les radicaux alkyle et aryle éventuellement substitués.

Selon un mode de réalisation particulier de l'invention, on utilise un acide carboxylique de formule $R_4$ COOH où $R_4$ représente un radical alkyle acyclique ou cyclique ayant de 1 à 12 atomes de carbone. On peut citer, en particulier, les acides

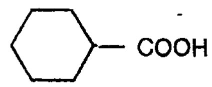 et $(CH_3)_3$ CCOOH

Selon un autre mode de réalisation particulier, on utilise un acide carboxylique de formule $R_4$ COOH dans laquelle $R_4$ représente un radical choisi parmi le groupe comprenant

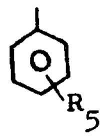 et 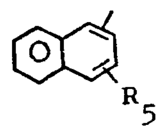

où $R_5$ représente un radical choisi parmi le groupe comprenant l'hydrogène, OH et les radicaux alkyle ayant de 1 à 12 atomes de carbone.

On peut citer en particulier les acides

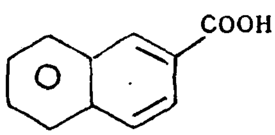 ; 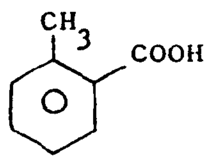 ; 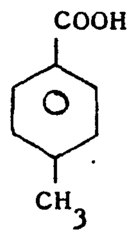 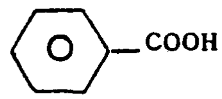 ; et 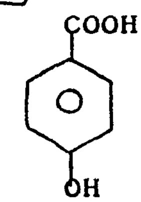

On opère généralement à une température comprise entre environ − 5 °C et environ 150 °C. La température est de préférence comprise entre environ 10 °C et 35 °C lorsqu'on opère sans ajouter d'acide carboxylique. Lorsqu'on opère en présence de ce dernier, la température est de préférence comprise entre 50 °C et 100 °C.

L'utilisation d'acide carboxylique, augmentant la stabilité du système catalytique, permet la mise en œuvre de la réaction à une température plus élevée ; il en résulte une augmentation de la vitesse de réaction et donc une augmentation de la productivité en alcool.

Le rapport molaire du butadiène à l'eau est de préférence compris entre environ 0,1 et 10. On préfère plus particulièrement utiliser le butadiène et l'eau dans un rapport molaire voisin de 1. Le rapport nombre de moles de butadiène au nombre d'atomes gramme de palladium est compris généralement entre 100 environ et 1 000 environ. On préfère utiliser une valeur voisine de 300. Des valeurs inférieures ou supérieures à cette fourchette ne sont pas exclues car elles n'agissent pas sur la sélectivité mais sur la vitesse de réaction. Pour obtenir la meilleure sélectivité, on opère de préférence avec un rapport molaire de l'eau à l'acide borique $B(OH)_3$ compris entre environ 0,1 et environ 20. Encore plus préférentiellement on utilise un rapport molaire $H_2O/B(OH)_3$ compris entre environ 2 et environ 10.

Le rapport du nombre de moles d'acide carboxylique au nombre d'atomes-gramme de palladium est, de préférence, compris entre environ 0,5 et environ 20. Encore plus préférentiellement il est compris entre environ 2 et environ 6.

On préfère mettre en œuvre le procédé selon l'invention en milieu homogène. On utilisera donc l'acide borique en quantité n'excédant pas la limite de solubilité (250 g/l de solvant environ à température ambiante). Les quantités d'acide borique sont donc déterminées par les quantités de solvant. Cette dernière doit être suffisante pour que le butadiène soit soluble dans le mélange eau-solvant.

On opère généralement à pression autogène, bien que des pressions supérieures ou inférieures à la pression autogène ne soient pas exclues du cadre de l'invention.

La durée de réaction est de préférence, lorsqu'on opère dans les fourchettes de températures préférentielles précitées, comprise entre 1 et 30 heures environ. Au-delà de 30 heures, la formation de lourds se développe.

On peut, pour diminuer la formation de lourds (oligomères du butadiène, polymères non fonctionnels et polymères possédant des fonctions alcools) ajouter dans le système réactionnel une phosphine comme la triphénylphosphine $P(\varphi)_3$. On utilise une phosphine préférentiellement en quantité telle que le rapport atomique $\frac{P_3 +}{Pd}$ est compris entre environ 0,1 et 0,5 environ. De préférence ce rapport est voisin de 0,25.

Le procédé de l'invention peut être mis en œuvre de façon continue ou discontinue. Les produits obtenus répondant à la formule (A) peuvent être hydrogénés en alcools linéaires saturés de formule

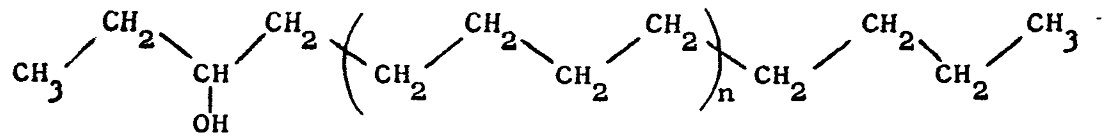

ou isomérisés et hydrogénés en alcools linéaires saturés de formule

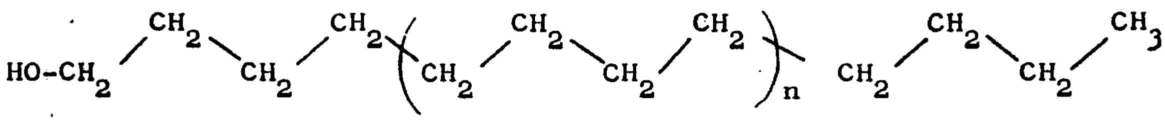

Ces alcools linéaires saturés sont des précurseurs de détergents biodégradables.

Les composés de formule A peuvent être aussi isomérisés en cétones de formule :

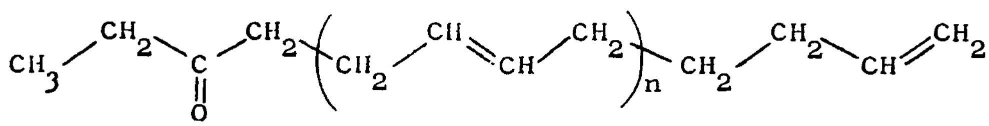

ou isomérisés et hydrogénés en cétones de formule

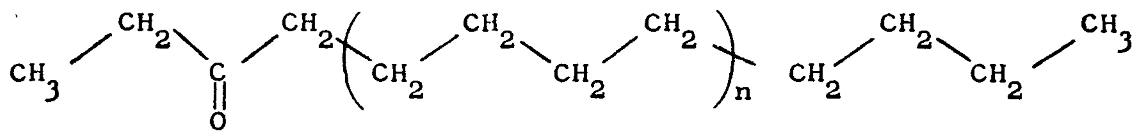

Le procédé selon l'invention est conduit de préférence, mais non obligatoirement, en atmosphère débarrassée d'oxygène.

D'autres caractéristiques et avantages de l'invention apparaîtront plus clairement à la lecture des exemples qui vont suivre. Ceux-ci ne sauraient être interprétés comme limitant de façon quelconque la portée de l'invention.

Exemples 1 à 8

Dans un tube en verre à parois épaisses dont l'agitation est assurée par un barreau téflonné entraîné par un agitateur magnétique, on introduit 80 mg d'acétate de palladium (0,35 milliatome-gramme de Pd) et 1 g (16 millimoles) d'acide borique : on purge trois fois par un système vide-argon et on introduit successivement 10 g de diméthylformamide, la quantité d'eau indiquée dans le tableau I et 4,5 g de butadiène (92 millimoles).

On agite pendant 24 h à 20 °C ; l'analyse par chromatographie en phase gazeuse montre qu'on obtient les composés suivants :

I 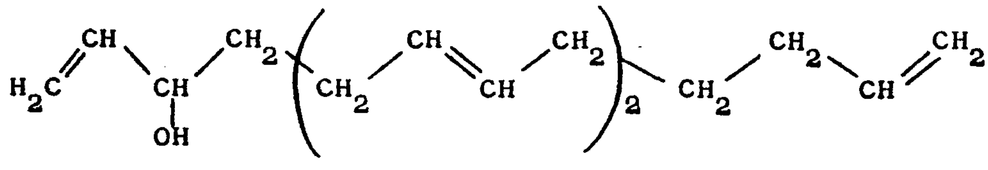

II 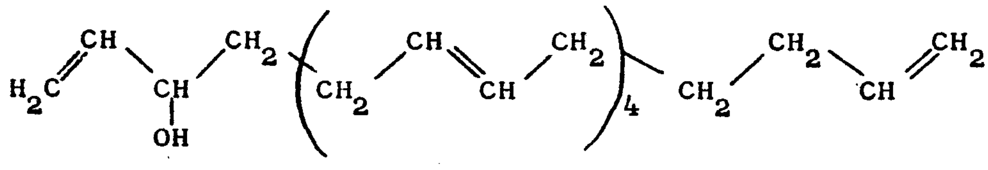

III produits d'oligomérisation du butadiène.

IV produits de polymérisation du butadiène ayant plus de 24 atomes de carbone.

Ces composés peuvent être séparés par toute technique bien connue de l'homme de l'art comme, par exemple, la distillation sous pression réduite.

Les résultats obtenus sont donnés dans le tableau I.

Exemples 9 à 14

On opère comme dans l'exemple 1 mais en faisant varier les qualités d'acide borique et d'eau comme indiqué dans le tableau II. Les résultats obtenus sont indiqués dans le tableau II.

Exemples 15-18

On opère comme dans l'exemple 1 mais en augmentant la quantité de solvant (diméthylformamide) de 10 à 15 g et en utilisant soit 1 g, soit 2 g d'acide borique avec des quantités variables d'eau comme indiqué dans le tableau III. Les résultats obtenus sont donnés dans le même tableau III.

Exemples 19-22

On opère comme dans l'exemple 1 avec un rapport molaire $H_2O/B(OH)_3 = 4$ et en utilisant les divers catalyseurs indiqués dans le tableau IV qui donne également les résultats obtenus.

Exemple 23

On opère comme dans l'exemple 1 en utilisant un rapport molaire $H_2O/B(OH)_3$ égal à 4 et ajoutant 29,6 mg de triphénylphosphine

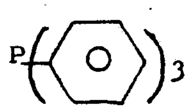

(0,113 milli at. g de $P^{3+}$).

Le taux de transformation du butadiène est 27,4 %.

On obtient :

I : 0,560 g
II : 0,095 g
III : 0,142 g
IV : 0,323 g

Exemple 24

On opère comme dans l'exemple 23 mais en ajoutant au lieu de triphénylphosphine 57,8 mg de triphénylphosphine métasulfonée

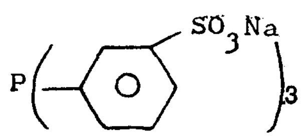 (0,094 milli at.g de $P^{3+}$)

Le taux de transformation du butadiène est de 34,2 %.

On obtient :

I : 0,629 g
II : 0,149 g
III : 0,161 g
IV : 0,400 g

Exemples 25-27

On opère comme dans l'exemple 1 avec un rapport $H_2O/B(OH)_3 = 4$ mais en faisant varier la nature du solvant (on utilise dans chaque cas 10 g de solvant). Les résultats sont donnés dans le tableau V.

Exemples 28-34

On opère comme dans l'exemple 1 avec un rapport molaire $H_2O/B(OH)_3 = 4$ mais en faisant varier la quantité d'acétate de palladium. Les résultats sont donnés dans le tableau VI.

Exemples 35-37

On opère comme dans l'exemple 1 avec un rapport molaire $H_2O/B(OH)_3 = 4$ mais en faisant varier les températures. Les résultats sont donnés dans le tableau VII.

Exemples 38-40

On opère comme dans l'exemple 1 avec un rapport molaire $H_2O/B(OH)_3 = 4$ mais en faisant varier la concentration en butadiène. Les résultats sont donnés dans le tableau VIII.

Exemples 41-50

On opère comme dans l'exemple 1 en utilisant un rapport molaire $H_2O/B(OH)_3$ soit égal à 2 (tableau IX, exemples 41 à 45) soit égal à 4 (tableau X, exemples 46 à 50) et en faisant varier les temps de réaction.

Exemple 51

Dans un autoclave en acier inoxydable de 0,5 l on introduit : 1,20 g (5,34 milli at.g de Pd) d'acétate de palladium et 15 g (242 millimoles) d'acide orthoborique. On purge trois fois par un système vide-argon et on introduit successivement 164 g de diméthylformamide, 17,64 g d'eau (0,98 mole) et 62 g de butadiène (1,15 mole). On agite à 20 °C pendant 24 h. Le taux de transformation du butadiène est de 41 %. Après addition d'eau et neutralisation de l'acide borique par du bicarbonate de sodium on recueille 27 g de phase organique dont l'analyse par chromatographie en phase gazeuse révèle qu'elle contient :

I : 10,08 g
II : 3 g
III : 1,31 g
IV : 11,29 g

Exemples 52 à 58

Dans un tube en verre à parois épaisses dont l'agitation est assurée par un barreau téflonné entraîné par un agitateur magnétique, on introduit 80 mg d'acétate de palladium (0,35 milliatome-gramme de Pd), 1 g (16 millimoles) d'acide orthoborique et une quantité d'acide carboxylique telle que le rapport atomique $\frac{H^+}{Pd}$ soit égal à 2 ; on purge trois fois par un système vide-argon et on introduit successivement 10 g de diméthylformamide, 1,2 g d'eau (rapport molaire $\frac{H_2O}{B(OH)_3} = 4$) et 4,5 g de butadiène (92 millimoles).

On agite pendant 24 h à 25 °C.
Les résultats obtenus sont indiqués dans les tableaux XI, XI'.

Exemple 59

On opère comme dans l'exemple 1 en utilisant :
$Pd(\emptyset\ COO)_2$ = 0,136 g (0,36 milliatome-g de Pd)
$B(OH)_3$ = 1 g
Diméthylformamide = 9,3 g
$H_2O$ = 1,18 g
Butadiène = 4,6 g
On chauffe à 50 °C pendant 2 h.
Le taux de transformation du butadiène est de 37,7 %.
On obtient :
I : 0,807 g
II : 0,267 g
III : 0,139 g
IV : 0,501 g

Exemple 60

On opère comme dans l'exemple 59 mais en chauffant à 50 °C pendant 16 h.
Le taux de transformation du butadiène est de 63,4 %.
On obtient :
I : 1,140 g
II : 0,333 g
III : 0,239 g
IV : 0,866 g

Exemple 61

On opère comme dans l'exemple 60 mais en utilisant 1,79 g d'$H_2O$.
Le taux de transformation du butadiène est de 54,8 %.
On obtient :
I : 1,325 g
II : 0,296 g
III : 0,240 g
IV : 0,305 g

Exemple 62

On opère comme dans l'exemple 1 en utilisant :
$Pd(AcO)_2$ : 81,6 mg (0,36 milli at.-g de Pd)
Acide benzoïque : 88 mg (0,72 millimole)
$B(OH)_3$ : 1 g
Diméthylformamide : 9,6 g
$H_2O$ : 1,23 g
Butadiène : 4,7 g
On chauffe à 50 °C pendant 2 h.
Le taux de transformation du butadiène est de 34 %.
On obtient :
I : 0,815 g
II : 0,206 g
III : 0,186 g
IV : 0,335 g

Exemple 63

On opère comme dans l'exemple 62 mais en utilisant 1,78 g d'$H_2O$.
Le taux de transformation du butadiène est de 21,3 %.
On obtient :
I : 0,506 g
II : 0,111 g
III : 0,162 g
IV : 0,149 g

Exemple 64

Dans un autoclave en acier inoxydable de 125 ml, on introduit :
$Pd(AcO)_2$ = 0,238 g (1,06 milli at.-g de Pd)
Acide benzoïque = 0,263 g (2,15 millimoles)
$B(OH)_3$ = 3 g
Diméthylformamide = 28,5 g
$H_2O$ = 3,6 g
Butadiène = 16 g
On chauffe sous agitation à 65 °C pendant 1 h.
Le taux de transformation du butadiène est de 30,4 %.
On obtient :
I : 2,292 g
II : 0,693 g
III : 0,714 g
IV : 0,918 g

Exemple 65

On opère comme dans l'exemple 1 en utilisant :
$Pd(AcO)_2$ = 0,080 g (0,35 milli at.-g de Pd)
Acide benzoïque = 0,187 g (1,53 millimole)
$B(OH)_3$ = 1 g
Diméthylformamide = 9,7 g
$H_2O$ = 1,20 g
Butadiène = 4,6 g
On chauffe à 50 °C pendant 4 h.
Le taux de transformation du butadiène est de 25,8 %.

On obtient :
I : 0,702 g
II : 0,118 g
III : 0,159 g
IV : 0,156 g

Exemple 66

On opère comme dans l'exemple 65 mais en chauffant à 50 °C pendant 16 h.
Le taux de transformation du butadiène est de 55,6 %.
On obtient :
I : 1,333 g
II : 0,167 g
III : 0,286 g
IV : 0,393 g

Exemple 67

On opère comme dans l'exemple 66 mais en utilisant 1,8 g d'$H_2O$.
Le taux de transformation du butadiène est de 35 %.
On obtient :
I : 0,921 g
II : 0,08 g
III : 0,161 g
IV : 0 g

Exemple 68

On opère comme dans l'exemple 67 mais en chauffant à 65 °C pendant 2 h.
Le taux de transformation du butadiène est de 29,1 %.
On obtient :
I : 0,757 g
II : 0,110 g
III : 0,193 g
IV : 0,035 g

Exemple 69

On opère comme dans l'exemple 64 avec :
$Pd(AcO)_2$ : 0,162 g (0,724 milli at.-g de Pd)
Acide benzoïque : 0,361 g (2,96 millimoles)
$B(OH)_3$ : 2 g
Diméthylformamide : 19 g
$H_2O$ : 3,6 g
Butadiène : 10 g
On chauffe à 80 °C pendant 1 h.
Le taux de transformation est de 31,4 %.
On obtient :
I : 1,59 g
II : 0,254 g
III : 0,572 g
IV : 0,230 g

Exemple 70

On opère comme dans l'exemple 69 mais en utilisant 0,110 g d'acétate de palladium (0,49 milli at.-g de Pd) et 0,243 g d'acide benzoïque (2 millimoles).
Le taux de transformation du butadiène est de 27,1 %.
On obtient :
I : 1,196 g
II : 0,190 g
III : 0,335 g
IV : 0,097 g

Exemple 71

On opère comme dans l'exemple 64 avec :
Pd(AcO)$_2$ : 0,166 g (0,74 milli at.-g de Pd)
Acide benzoïque : 0,361 g (2,96 millimoles)
$B(OH)_3$ : 4 g
Diméthylformamide : 28,5 g
$H_2O$ : 7 g
Butadiène : 11 g
On chauffe sous agitation à 95 °C pendant 30 mn.
Le taux de transformation du butadiène est de 44,1 %.
On obtient :
I : 1,807 g
II : 0,413 g
III : 1,179 g
IV : 0,413 g

Exemple 72

On opère comme dans l'exemple 71 mais en utilisant 0,152 g d'acétate de palladium (0,68 milli at.-g de Pd), 0,334 g d'acide benzoïque (2,73 millimoles) et 14 g de butadiène.
On chauffe à 80 °C pendant 1 h.
Le taux de transformation du butadiène est de 29,1 %.
On obtient :
I : 2,06 g
II : 0,242 g
III : 0,643 g
IV : 0,428 g

TABLEAU I

| Exemples | Quantité d'eau (g) | Rapport molaire $H_2O/B(OH)_3$ | Taux de transformation du butadiène - (%) | Produits obtenus (g) I | II | III | IV |
|---|---|---|---|---|---|---|---|
| 1 | 0 | 0 | 3,5 | 0 | 0 | 0,1 | 0 |
| 2 | 0,14 | 0,48 | 11,5 | 0,230 | 0,058 | 0,079 | 0,112 |
| 3 | 0,29 | 1 | 30,3 | 0,481 | 0,2 | 0,095 | 0,605 |
| 4 | 0,62 | 2,1 | 34,9 | 0,413 | 0,22 | 0,113 | 0,875 |
| 5 | 1,09 | 3,65 | 34 | 0,572 | 0,22 | 0,084 | 0,668 |
| 6 | 1,2 | 4,2 | 31,9 | 0,514 | 0,157 | 0,081 | 0,64 |
| 7 | 1,85 | 6,5 | 28,7 | 0,573 | 0,133 | 0,130 | 0,49 |
| 8 | 2,8 | 9,45 | 21 | 0,397 | 0,13 | 0,119 | 0,24 |

TABLEAU II

| Exemples | Quantité de $B(OH)_3$ (g) | Quantité d'eau (g) | Rapport molaire $H_2O/B(OH)_3$ | Taux de transformation du butadiène (%) | Produits obtenus (g) I | II | III | IV |
|---|---|---|---|---|---|---|---|---|
| 9 | 0 | 0 | 0 | 0 | 0 | 0 | 0 | 0 |
| 10 | 0,5 | 0,3 | 2.04 | 32 | 0,192 | 0,150 | 0,10 | 1 |
| 11 | 1,01 | 0,55 | 1,86 | 46,7 | 0,832 | 0,268 | 0,10 | 1,05 |

TABLEAU II (suite)

| Exemples | Quantité de $B(OH)_3$ (g) | Quantité d'eau (g) | Rapport molaire $H_2O/B(OH)_3$ | Taux de transformation du butadiène (%) | Produits obtenus (g) | | | |
|---|---|---|---|---|---|---|---|---|
| | | | | | I | II | III | IV |
| 12 | 2,01 | 1,34 | 2,28 | 23 | 0,695 | 0,160 | 0,02 | 0,30 |
| 13 | 2,09 | 2,36 | 3,9 | 15,4 | 0,36 | 0,091 | 0,12 | 0,246 |
| 14 | 1,96 | 3,43 | 6 | 15,9 | 0,358 | 0,018 | 0,08 | 0,17 |

TABLEAU III

| Exemple | Quantité $B(OH)_3$ (g) | Quantité $H_2O$ (g) | Rapport molaire $H_2O/B(OH)_3$ | Taux de transformation du butadiène (%) | Produits obtenus (g) | | | |
|---|---|---|---|---|---|---|---|---|
| | | | | | I | II | III | IV |
| 15 | 1 | 1,1 | 3,7 | 25 | 0,197 | 0,06 | 0,026 | 0,757 |
| 16 | 1 | 1,67 | 5,5 | 35,7 | 0,612 | 0,164 | 0,056 | 0,680 |
| 17 | 2 | 2,49 | 4,2 | 21,8 | 0,533 | 0,136 | 0,029 | 0,330 |
| 18 | 2 | 3,73 | 6,7 | 22,7 | 0,663 | 0,090 | 0,088 | 0,266 |

TABLEAU IV

| Exemple | Catalyseur | | Nombre de milliatome gramme de Pd | Taux de transformation du butadiène % | Produits obtenus (g) | | | |
|---|---|---|---|---|---|---|---|---|
| | Nature | Quantité (g) | | | I | II | III | IV |
| 19 | $Pd(CH_3\text{-}COO)_2$ | 0,08 | 0,35 | 47,0 | 0,897 | 0,271 | 0,090 | 0,944 |
| 20 | 1) | 0,107 | 0,4 | 55 | 0,392 | 0,264 | 0,037 | 1,48 |
| 21 | 2) | 0,063 | 0,306 | 27,8 | 0,486 | 0,130 | 0,024 | 1,10 |
| 22 | $(\varphi\text{-}COO)_2Pd$ | 0,121 | 0,325 | 37,2 | 0,943 | 0,221 | 0,094 | 0,419 |

1) 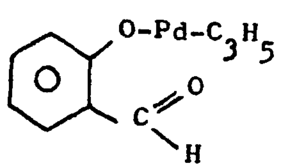 : salicylate de palladium π allyle,

2) $(CH_3\text{-}COOPdC_3H_5)_2$ : bis (π-allyle palladium acétate).

TABLEAU V

| Exemple | Nature du solvant | Taux de transformation du butadiène (%) | Produits obtenus (g) | | | |
|---|---|---|---|---|---|---|
| | | | I | II | III | IV |
| 25 | Diméthylformamide | 47 | 0,897 | 0,271 | 0,090 | 0,944 |
| 26 | N.méthylpyrrolidone | 27,4 | 0,522 | 0,101 | 0,104 | 0,408 |
| 27 | Carbonate de propylène | 5 | 0,028 | 0,045 | 0,010 | 0,355 |

TABLEAU VI

| Exemple | Concentration en Pd p.p.m. | Quantité d'acétate de Pd (mg) | Nombre de milli at-g de Pd | Taux de transformation du butadiène (%) | Produits obtenus (g) | | | |
|---|---|---|---|---|---|---|---|---|
| | | | | | I | II | III | IV |
| 28 | 760 | 26,5 | 0,118 | 10,7 | 0,230 | 0,045 | 0,018 | 0,175 |
| 29 | 1 320 | 46,5 | 0,207 | 23,4 | 0,456 | 0,116 | 0,046 | 0,39 |
| 30 | 2 250 | 79,5 | 0,35 | 47 | 0,897 | 0,271 | 0,090 | 0,944 |
| 31 | 3 000 | 105,6 | 0,470 | 58,3 | 1,034 | 0,343 | 0,139 | 1,164 |
| 32 | 4 000 | 140,8 | 0,627 | 68,3 | 1,198 | 0,414 | 0,192 | 1,315 |
| 33 | 4 500 | 158 | 0,705 | 74,4 | 1,159 | 0,468 | 0,212 | 1,414 |
| 34 | 5 070 | 180 | 0,800 | 77,2 | 1,216 | 0,468 | 0,253 | 1,653 |

TABLEAU VII

| Exemple | Température (°C) | Taux de transformation du butadiène (%) | Produits obtenus (g) | | | |
|---|---|---|---|---|---|---|
| | | | I | II | III | IV |
| 35 | 10 | 11 | 0,139 | 0,036 | 0,020 | 0,315 |
| 36 | 35 | 63,2 | 1,089 | 0,354 | 0,231 | 1,263 |
| 37 | 50 | 66,6 | 0,771 | 0,419 | 0,323 | 1,491 |

TABLEAU VIII

| Exemple | Quantité de butadiène (g) | Taux de transformation du butadiène (%) | Produits obtenus (g) | | | |
|---|---|---|---|---|---|---|
| | | | I | II | III | IV |
| 38 | 2,48 | 36,8 | 0,592 | 0,151 | 0,080 | 0,450 |
| 39 | 4,5 | 31,9 | 0,514 | 0,157 | 0,081 | 0,638 |
| 40 | 9,65 | 12,6 | 0,500 | 0,150 | 0,077 | 0,520 |

TABLEAU IX

| Exemples | Temps de réaction (h) | Taux de transformation du butadiène (%) | Produits obtenus (g) | | | |
|---|---|---|---|---|---|---|
| | | | I | II | III | IV |
| 41 | 5 | 13,2 | 0,235 | 0,090 | 0,043 | 0,38 |
| 42 | 7 | 25,9 | 0,351 | 0,154 | 0,055 | 0,48 |
| 43 | 17 | 39 | 0,972 | 0,220 | 0,083 | 0,56 |
| 44 | 41 | 51 | 0,665 | 0,254 | 0,110 | 0,823 |
| 45 | 95 | 54,7 | 0,405 | 0,364 | 0,130 | 1,56 |

TABLEAU X

| Exemples | Temps de réaction (h) | Taux de transformation du butadiène (%) | Produits obtenus (g) | | | |
|---|---|---|---|---|---|---|
| | | | I | II | III | IV |
| 46 | 7 | 12,6 | 0,23 | 0,075 | 0,030 | 0,260 |
| 47 | 16 | 26,5 | 0,47 | 0,140 | 0,050 | 0,520 |
| 48 | 24 | 31,9 | 0,51 | 0,157 | 0,081 | 0,638 |
| 49 | 40 | 43 | 0,833 | 0,310 | 0,170 | 0,960 |
| 50 | 96 | 48,7 | 0,861 | 0,340 | 0,200 | 1,050 |

TABLEAU XI

| Exemples | Acide ajouté | | Taux de transformation du butadiène (%) | Produits obtenus (g) | | | |
|---|---|---|---|---|---|---|---|
| | Type | Masse (mg) | | I | II | III | IV |
| 52 | 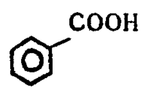 | 87,2 | 27,4 | 0,679 | 0,124 | 0,074 | 0,31 |
| 53 | 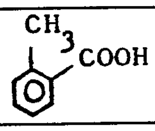 | 98 | 30 | 0,684 | 0,150 | 0,103 | 0,228 |
| 54 | 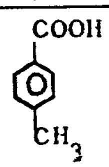 | 95 | 25,2 | 0,636 | 0,098 | 0,052 | 0,333 |
| 55 | 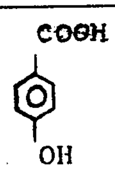 | 94 | 31,1 | 0,770 | 0,159 | 0,074 | 0,453 |

TABLEAU XI'

| Exemples | Acide ajouté | | Taux de transformation du butadiène (%) | Produits obtenus (g) | | | |
|---|---|---|---|---|---|---|---|
| | Type | Masse (mg) | | I | II | III | IV |
| 56 | 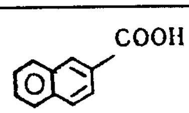 | 130 | 11 | 0,266 | 0,029 | 0,075 | 0,069 |
| 57 | 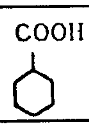 | 93 | 32,3 | 0,705 | 0,200 | 0,151 | 0,491 |
| 58 | 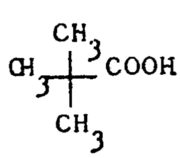 | 82 | 29,5 | 0,535 | 0,103 | 0,081 | 0,595 |

**Revendications**

1. Alcools gras insaturés de formule :

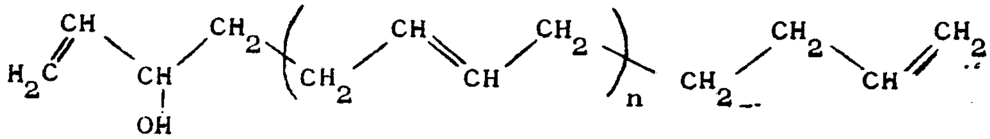

dans laquelle
n = 2 ou 4.

2. Hexadécatétraène-1,6,10,15 ol-3.

3. Tétracosahexaène-1,6,10,14,18,23 ol-3.

4. Procédé de préparation d'alcools gras insaturés selon la revendication 1, caractérisé en ce que l'on fait réagir du butadiène avec de l'eau en présence d'un sel de palladium et d'acide borique $B(OH)_3$ dans un solvant aprotique polaire.

5. Procédé selon la revendication 4, caractérisé en ce que l'on fait réagir le butadiène avec l'eau en présence, en outre, d'un acide carboxylique.

6. Procédé selon la revendication 4, caractérisé en ce que le sel de palladium est choisi parmi le groupe comprenant les carboxylates de palladium de formule $(R_1\,COO)_2Pd$, les complexes allyliques du palladium possédant un radical carboxylate de formule $(R_2\,Pd\,OOCR_1)_2$ et les complexes allyliques du palladium de formule $R_2\,Pd\,OR_1$
où
$R_1$ représente un radical choisi parmi le groupe comprenant les radicaux alkyle ayant de 1 à 6 atomes de carbone et les radicaux aryle, ces radicaux pouvant être éventuellement substitués,
$R_2$ représente un radical de formule :

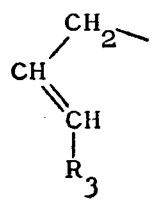

où
$R_3$ représente un atome d'hydrogène ou le radical $R_1$.

7. Procédé selon la revendication 6, caractérisé en ce que le sel de palladium est l'acétate de palladium ou le benzoate de palladium.

8. Procédé selon la revendication 6, caractérisé en ce que le sel de palladium est choisi parmi le groupe comprenant :

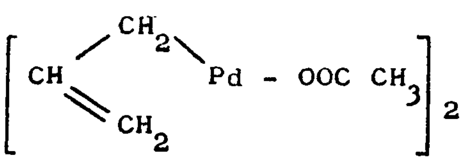

;

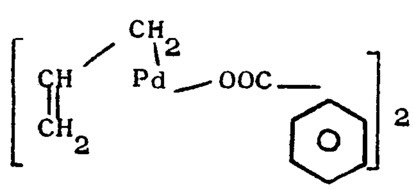

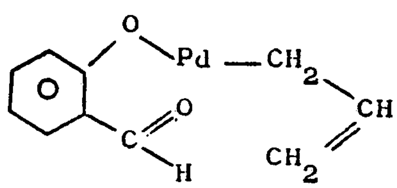

9. Procédé selon la revendication 4, caractérisé en ce que le solvant est choisi parmi le groupe comprenant la diméthylformamide, l'hexaméthyl-phosphoretriamide, la diméthylacétamide, la N-méthylpyrrolidone.

10. Procédé selon la revendication 5, caractérisé en ce que l'acide carboxylique a pour formule générale :

$$R_4\,COOH$$

dans laquelle $R_4$ représente un radical choisi parmi le groupe comprenant les radicaux alkyle et aryle éventuellement substitués.

11. Procédé selon la revendication 10, caractérisé en ce que $R_4$ est choisi parmi le groupe comprenant les radicaux alkyle acycliques et cycliques ayant de 1 à 12 atomes de carbone.

12. Procédé selon la revendication 11, caractérisé en ce que $R_4$ représente

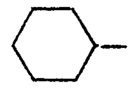 ou $(CH_3)_3C$—

13. Procédé selon la revendication 9, caractérisé en ce que $R_4$ représente un radical choisi parmi le groupe comprenant :

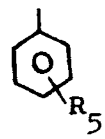 et 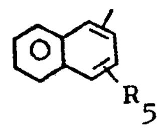

où $R_5$ représente au moins un radical choisi parmi le groupe comprenant l'hydrogène, OH et les radicaux alkyle ayant de 1 à 12 atomes de carbone.

14. Procédé selon la revendication 13, caractérisé en ce que $R_5$ représente l'hydrogène.

15. Procédé selon l'une quelconque des revendications précédentes caractérisé en ce qu'on opère à une température comprise entre – 5 °C et 150 °C.

16. Procédé selon la revendication 4, caractérisé en ce que le rapport molaire du butadiène à l'eau est compris entre environ 0,1 et 10.

17. Procédé selon la revendication 16, caractérisé en ce que le rapport molaire est égal à environ 1.

18. Procédé selon la revendication 4, caractérisé en ce que le rapport du nombre de moles de butadiène au nombre d'at.-g de palladium est compris entre environ 100 et environ 1 000.

19. Procédé selon la revendication 18, caractérisé en ce que le rapport est égal à environ 300.

20. Procédé selon la revendication 4, caractérisé en ce que le rapport molaire de l'eau à l'acide borique est compris entre environ 0,1 et environ 20.

21. Procédé selon la revendication 20, caractérisé en ce que le rapport est compris entre environ 2 et environ 10.

22. Procédé selon la revendication 5, caractérisé en ce que le rapport du nombre de moles d'acide carboxylique au nombre d'atomes-gramme de palladium est compris entre environ 0,5 et environ 20.

23. Procédé selon la revendication 22, caractérisé en ce que le rapport est compris entre environ 2 et environ 6.

24. Procédé selon l'une quelconque des revendications 4 à 23, caractérisé en ce qu'on introduit dans le milieu réactionnel une phosphine.

25. Procédé selon la revendication 24, caractérisé en ce que la phosphine est précédente en quantité telle que le rapport atomique $\frac{P^{3+}}{Pd}$ est compris entre environ 0,1 et environ 0,5.

**Claims**

1. Unsaturated fatty alcohols of the formula :

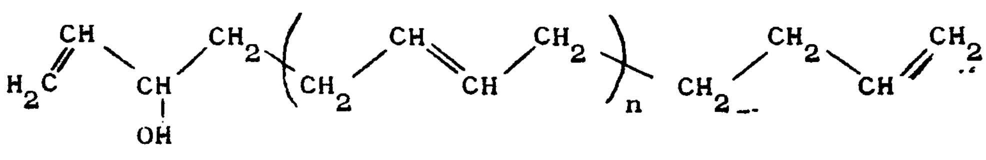

in which

n = 2 or 4.

2. Hexadeca-1,6,10,15-tetraen-4-ol.

3. Tetracosa-1,6,10,14,18,23-hexaen-3-ol.

4. Process for the preparation of unsaturated fatty alcohols according to Claim 1, characterised in that butadiene is reacted with water, in the presence of a palladium salt and boric acid, $B(OH)_3$, in a polar aprotic solvent.

5. Process according to Claim 4, characterised in that the reaction of butadiene with water is also carried out in the presence of a carboxylic acid.

6. Process according to Claim 4, characterised in that the palladium salt is chosen from amongst the group comprising the palladium carboxylates of the formula $(R_1COO)_2$ Pd, the allyl complexes of palladium possessing a carboxylate radical, of the formula $(R_2PdOOCR_1)_2$, and the allyl complexes of palladium of the formula $R_2PdOR_1$,

in which

$R_1$ represents a radical chosen from amongst the group comprising alkyl radicals having from 1 to 6 carbon atoms and aryl radicals, it being possible for these radicals to be optionally substituted, and

$R_2$ represents a radical of the formula :

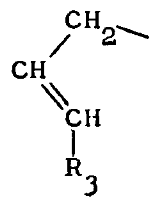

in which

$R_3$ represents a hydrogen atom or the radical $R_1$.

7. Process according to Claim 6, characterised in that the palladium salt is palladium acetate or palladium benzoate.

8. Process according to Claim 6, characterised in that the palladium salt is chosen from amongst the group comprising :

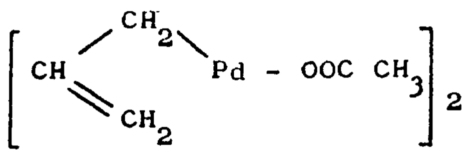 ; 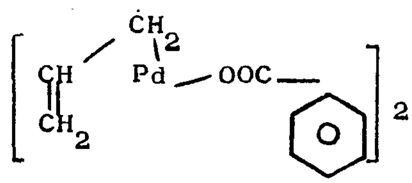

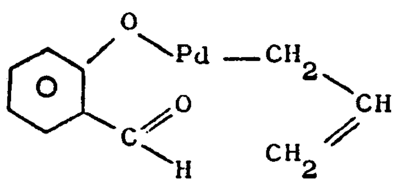

9. Process according to Claim 4, characterised in that the solvent is chosen from amongst the group comprising dimethylformamide, hexamethylphosphorotriamide, dimethylacetamide and N-methylpyrrolidone.

10. Process according to Claim 5, characterised in that the carboxylic acid has the general formula :

$$R_4COOH$$

in which $R_4$ represents a radical chosen from amongst the group comprising optionally substituted alkyl and aryl radicals.

11. Process according to Claim 10, characterised in that $R_4$ is chosen from amongst the group comprising acyclic and cyclic alkyl radicals having from 1 to 12 carbon atoms.

12. Process according to Claim 11, characterised in that $R_4$ represents

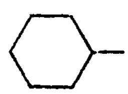 or $(CH_3)_3C$—.

13. Process according to Claim 9, characterised in that $R_4$ represents a radical chosen from amongst the group comprising :

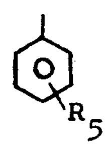 and 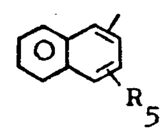

in which $R_5$ represents at least one radical chosen from amongst the group comprising hydrogen, OH and alkyl radicals having from 1 to 12 carbon atoms.

14. Process according to Claim 13, characterised in that $R_5$ represents hydrogen.

15. Process according to any one of the preceding claims, characterised in that the reaction is carried out at a temperature between − 5 °C and 150 °C.

16. Process according to Claim 4, characterised in that the molar ratio of the butadiene to the water is between about 0.1 and 10.

17. Process according to Claim 16, characterised in that the molar ratio is equal to about 1.

18. Process according to Claim 4, characterised in that the ratio of the number of mols of butadiene to the number of g atoms of palladium is between about 100 and about 1 000.

19. Process according to Claim 18, characterised in that the ratio is equal to about 300.

20. Process according to Claim 4, characterised in that the molar ratio of the water to the boric acid is between about 0.1 and about 20.

21. Process according to Claim 20, characterised in that the ratio is between about 2 and about 10.

22. Process according to Claim 5, characterised in that the ratio of the number of mols of carboxylic acid to the number of gram atoms of palladium is between about 0.5 and about 20.

23. Process according to Claim 22, characterised in that the ratio is between about 2 and about 6.

24. Process according to any one of Claims 4 to 23, characterised in that a phosphine is introduced into the reaction medium.

25. Process according to Claim 24, characterised in that the phosphine is present in an amount such that the atomic ratio $\frac{P^{3+}}{Pd}$ is between about 0.1 and about 0.5.

**Ansprüche**

1. Ungesättigte Fettalkohole der Formel

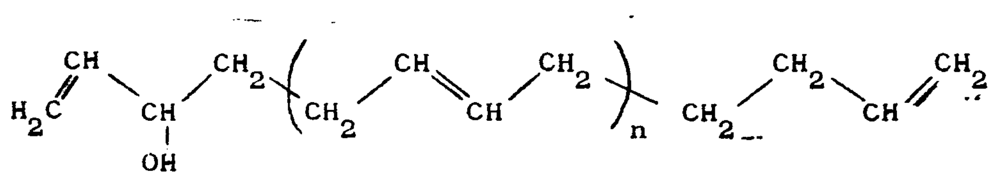

worin

n = 2 oder 4 ist.

2. Hexadeca-1,6,10,15-tetraen-3-ol.

3. Tetracosa-1,6,10,14,18,23-hexaen-3-ol.

4. Verfahren zur Herstellung von ungesättigten Fettalkoholen gemäß Anspruch 1, dadurch gekennzeichnet, daß man Butadien mit Wasser in Gegenwart eines Palladiumsalzes und von Borsäure $B(OH)_3$ in einem aprotischen polaren Lösungsmittel zur Reaktion bringt.

5. Verfahren gemäß Anspruch 4, dadurch gekennzeichnet, daß man das Butadien mit Wasser außerdem in Gegenwart einer Carbonsäure zur Reaktion bringt.

6. Verfahren gemäß Anspruch 4, dadurch gekennzeichnet, daß das Palladiumsalz ausgewählt ist aus der Gruppe umfassend die Palladiumcarboxylate der Formel $(R_1COO)_2$ Pd, die Allylkomplexe des Palladiums, welche einen Carboxylatrest der Formel $(R_2\ Pd\ OOCR_1)_2$ aufweisen und die Allylkomplexe des Palladiums der Formel $R_2$ Pd $OR_1$,

worin

$R_1$ einen Rest ausgewählt aus der Gruppe, umfassend die Alkylreste mit 1 bis 6 Kohlenstoffatomen und die Arylreste bedeutet, wobei diese Reste auch gegebenenfalls substituiert sein können,

$R_2$ einen Rest der Formel

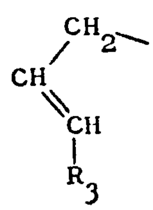

bedeutet, wobei

$R_3$ ein Wasserstoffatom oder den Rest $R_1$ bedeutet.

7. Verfahren gemäß Anspruch 6, dadurch gekennzeichnet, daß das Palladiumsalz das Palladiumacetat oder das Palladiumbenzoat ist.

8. Verfahren gemäß Anspruch 6, dadurch gekennzeichnet, daß das Palladiumsalz ausgewählt ist aus der Gruppe, umfassend

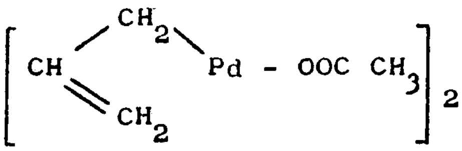 ; 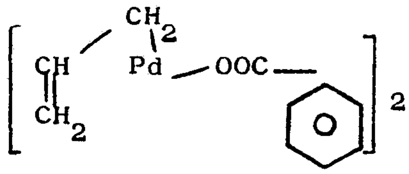

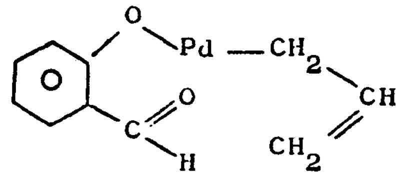

9. Verfahren gemäß Anspruch 4, dadurch gekennzeichnet, daß das Lösungsmittel ausgewählt ist aus der Gruppe, umfassend Dimethylformamid, Hexamethylphosphortriamid, Dimethylacetamid, N-Methylpyrrolidon.

10. Verfahren gemäß Anspruch 5, dadurch gekennzeichnet, daß die Carbonsäure die allgemeine Formel

$$R_4COOH$$

hat, wobei $R_4$ einen Rest ausgewählt aus der Gruppe, umfassend die Alkyl- und Arylreste, gegebenenfalls substituiert bedeutet.

11. Verfahren gemäß Anspruch 10, dadurch gekennzeichnet, daß $R_4$ ausgewählt ist aus der Gruppe, umfassend die acyclischen und cyclischen Alkylreste mit 1 bis 12 Kohlenstoffatomen.

12. Verfahren gemäß Anspruch 11, dadurch gekennzeichnet, daß $R_4$ bedeutet

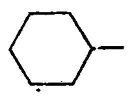 oder $(CH_3)_3C$—

13. Verfahren gemäß Anspruch 9, dadurch gekennzeichnet, daß $R_4$ einen Rest bedeutet, ausgewählt aus der Gruppe, umfassend

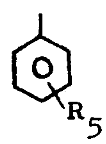 und 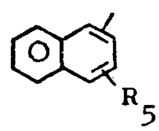

wobei $R_5$ wenigstens einen Rest bedeutet, ausgewählt aus der Gruppe, umfassend Wasserstoff, OH und die Alkylreste mit 1 bis 12 Kohlenstoffatomen.

14. Verfahren gemäß Anspruch 13, dadurch gekennzeichnet, daß $R_5$ Wasserstoff bedeutet.

15. Verfahren gemäß einem der vorhergehenden Ansprüche, dadurch gekennzeichnet, daß man bei einer Temperatur von zwischen − 5 °C und 150 °C arbeitet.

16. Verfahren gemäß Anspruch 4, dadurch gekennzeichnet, daß das Molverhältnis des Butadiens zu Wasser zwischen etwa 0,1 und 10 beträgt.

17. Verfahren gemäß Anspruch 16, dadurch gekennzeichnet, daß das Molverhältnis etwa gleich 1 ist.

18. Verfahren gemäß Anspruch 4, dadurch gekennzeichnet, daß das Verhältnis der Molzahl Butadien zur Anzahl Atom-g Palladium zwischen etwa 100 und etwa 1 000 beträgt.

19. Verfahren gemäß Anspruch 18, dadurch gekennzeichnet, daß das Verhältnis etwa 300 ist.

20. Verfahren gemäß Anspruch 4, dadurch gekennzeichnet, daß das Molverhältnis von Wasser zu Borsäure zwischen etwa 0,1 und etwa 20 beträgt.

21. Verfahren gemäß Anspruch 20, dadurch gekennzeichnet, daß das Verhältnis zwischen etwa 2 und etwa 10 beträgt.

22. Verfahren gemäß Anspruch 5, dadurch gekennzeichnet, daß das Verhältnis der Molzahl Carbonsäure zur Zahl g-Atome Palladium zwischen etwa 0,5 und etwa 20 liegt.

23. Verfahren gemäß Anspruch 22, dadurch gekennzeichnet, daß das Verhältnis zwischen etwa 2 und etwa 6 beträgt.

24. Verfahren gemäß einem der Ansprüche 4 bis 23, dadurch gekennzeichnet, daß man in das Reaktionsmilieu ein Phosphin einführt.

25. Verfahren gemäß Anspruch 24, dadurch gekennzeichnet, daß das Phosphin in einer solchen Menge vorhanden ist, daß das Atomverhältnis $\frac{P^{3+}}{Pd}$ zwischen etwa 0,1 und etwa 0,5 beträgt.